# EUROPEAN PATENT APPLICATION

(11) **EP 3 269 342 A1**
(43) Date of publication of application: **17.01.2018**
(21) Application number: 16460045.4
(22) Date of filing: 12.07.2016
(51) Int. Cl.: A61G 1/04, A61G 1/044

(54) **A SYSTEM FOR TRANSPORTING AND IMMOBILIZING A PATIENT, IN PARTICULAR STROKE PATIENT FOR CT IMAGING**

(71) Applicant: Piasecki, Krzysztof, 35329 Rzeszow (PL)
(72) Inventor: Piasecki, Krzysztof, 35329 Rzeszow (PL)
(74) Representative: Karczmitowicz, Teresa Ewa

(57) **Abstract**

A system for transporting and immobilizing a patient, in particular stroke patient for CT imaging, comprises a stretcher (1) made of rigid radiolucent material, e.g. polycarbonate, with a body section (2) and a narrower head section (3), and a removable head rest (4) made of rigid radiolucent material, e.g. styrofoam, mounted in the head section of the stretcher. The stretcher (1) has the belt slits (10) for the immobilizing belts (9), shaped in the body side walls (6) and in the head side walls (7), and the handle slits (11) for transfer of the stretcher, shaped in the body side walls (6) and in the bottom (5). The immobilizing belts (9) comprise the velcro closures or plastic clips. The stretcher bottom (5) may be covered with an elastic liner (18). The head rest (4) consists of a head rest bottom part (12), and a head rest upper part (13), wherein the head rest bottom part (12) has a lower cavity (14) for the back of the patient's head and neck, and the head rest upper part (13) has an upper cavity (15) for the facial part of the patient's head, with an opening (16) for the patient's mouth, nose and eyes. The head rest bottom part (12) and the head rest upper part (13) can be joined by a velcro closure or plastic clips.

## Description

The present invention refers to transporting and immobilizing a patient, before and during medical imaging or radiotherapy, e.g. CT, NMR, EPR, and the like. In particular the invention enables the medical personnel to immobilize the patient's head quickly and surely. Time and precision are of great importance in case of a brain stroke, where the CT imaging has to performed as early as possible and with high definition to enable immediate diagnosis and therapy.

The patient should be transported quickly and safely, and placed directly onto the table of the CT device. Such a procedure helps to avoid additional movements of the patient's head relative to the body.

However, the known stretchers do not enable performing the CT of the patient's head without transferring him onto the CT device table. Usually the reason is very simple: the stretchers are wider than the gantry of the CT device and cannot be inserted into the gantry. Besides, the stretchers usually contain metallic parts which cannot be located in the gantry or its vicinity. Or, if the known stretchers can be placed on the table, the patient's body and head have to be additionally immobilized. This creates several problems.

Firstly, every minute lost for additional operations reduces the patient's chances for recovery, because the nervous cells die quickly in case of a damage inside the brain.

Secondly, any uncontrolled movement of the patient's head relative to the body can worsen his condition.

Thirdly, most of stroke patients are elderly people with obesity which makes difficult repositioning of the patient.

Fourthly, the majority of stroke patients loose conscience and are unable either to help the personnel, or to cooperate during the medical examination. They may also have seizures.

The problem arises how to overcome these obstacles. The proposed invention answers this problem and makes a meaningful step forward in relation to the state of the art which is analyzed below.

International patent application WO2016054407 A1 proposes a stretcher which comprises a bed, and a frame having first and second parallel frame members coupled to first and second lateral frame member. A securement track is defined by an upper surface of at least one of the frame members, having a plurality of alternating open regions and necked down regions along a length. A bed mount is configured to both support the bed and be releasably secured in the securement track in a secure condition, and to be repositionable along the length of the securement track in an unsecured condition.

American patent document US4566445 A discloses a stretcher for traumatized patients comprising a rigid radiolucent board having a central core of polyimide foam and a skin of graphite reinforced epoxy. The board has built in handles for transporting the patient, straps for holding a patient on the board, and means for applying traction to the patient. The stretcher is designed for transporting the patient and applying a variety of diagnostic tests, including computer aided tomography scanning, without removing the patient from the stretcher. It also facilitates efforts to make a closed reduction of a cervical fracture with fluoroscopic monitoring.

Techniques and devices for securing a medical device to a patient-carrying device, such as a mechanical cardiopulmonary resuscitation (CPR) device to a stretcher, are described in American patent application US2016095765 A1. A medical device stabilization strap includes a first removable attachment shackle connected to a first end of a first strap. An adjustable quick release buckle is disposed between a second end of the first strap and a proximal end of a second strap, while a second removable attachment shackle is connected to a distal end of the second strap. The first and second removable attachment shackles each includes a U-shaped bracket for removably engaging a medical device. The adjustable quick release buckle adjusts a length of the second strap, for example, to secure the medical device to the patient-carrying device.

Many inventions solve the problem of supporting and immobilizing the head relative to the body of the patient, and/or to the table of an imaging device.

According to American patent application US2007287943 A1 a spinal restraint device includes a body member with a head support portion, a neck support portion, and a back support portion. Left and right body wrap portions extend from the back support portion, and left and right head wrap portions extend from the neck support portion, and these portions respectively wrap around an individual's torso and head and are secured to each other to secure the torso and head against movement. A chest flap section is provided in the left and right body wrap portions for folding away from the individual's chest, for comfort or for better access to the chest area. Declaration buckles and straps are also provided for securing the spinal restraint device (and individual) to a support board or stretcher.

In WO2015109086 A1 an adapter to position and restrain a patient's head movement includes a base, one or more attachment members to selectively affix the base to an imaging table, a pair of upwardly extending spaced-apart supports attached to the base, a stabilizer frame rotatably attached to the pair of supports, and a locking mechanism to selectively prevent rotation of the frame attached to said pair of supports.

Other solutions for the head rest designed for the patient lying on the table are disclosed in Chinese documents CN203988099 A1, CN202005759 A1, or CN201398973 Y. The common feature of these solutions is that the head rest comprises a more or less rigid bolster or pillow for elevating the head above the table plane, and the immobilizing means like bands or shackles.

Similar ideas are realized also in American patents US5337760 A, or US5311882 A.

A variant of the head immobilizer is equipped with a head mask. For example European patent application EP2926735 A2 discloses a frame for securing a thermoplastic mask, which comprises a first support portion for the upper part of the patient's back and a second support portion for securing the patient's head in an upright position. The two support portions comprise means for anchoring the thermoplastic mask and securing it behind the patient's head.

Similar solutions are proposed in international patent application WO2011000077 A1 and Japanese patent document JPH09262231 A. An active apparatus for maintaining the position of a patient during a medical procedure such as imaging, also involving a head mask, is disclosed in American patent US6138302 A. The apparatus includes a system for tracking motion of the particular body portion of interest, such as the head, a computer for receiving signals from the tracking system, and determining if undesired motion of the body portion has occurred and, if so, issuing a corrective signal which through a plurality of motors effects repositioning of the body portion to the desired location. This monitoring and repositioning may take place at a very high frequency. A particular contemplated use is immobilizing and positioning of the head during brain scans.

In a number of inventions like the one described in international patent application WO2004075716 A2 the dental-based stereotactic localizers are disclosed. The localizer includes a dental piece configured and adapted to be received in the mouth of the patient, a support structure secured to the dental piece, the support structure defining an arcuate configuration which extends to the posterior margins of the teeth of the patient thus allowing an occipital region of the head of the patient to directly contact a surface of a treatment table. Additionally, a plurality of graphic reference elements produce index information on an image scan when intersected by a plane of a scan slice, wherein the index information is used to develop a mapping of data from the image scan so as to reference the data from the image scan to a coordinate system of the dental tray.

Dental impression means, as well as a graphic reference with fiducials such as points, rods, or diagonals that can reference a selected target position determined from tomographic imaging, are used also in American patents US5947981 A and US4841965 A. However, in spite of good immobilizing effect, the dental impressions usually cannot be used for stroke patients, who are often unconscious, or suffer from trembling or seizures.

Other related inventions of the head support are proposed in American patent documents US2010268248 A1, US5865780 A and US4841965 A mentioned above. The latter invention involves immobilizing the head with negative air pressure. The patient's head is placed in the cradle equipped with the holes leading to the chamber which is partially evacuated. The patient's head can be held against the rim by the vacuum.

None of the above described inventions solve the technical problem of providing the medical rescue teams with a stretcher suitable for placing it with safely secured patient directly on a table of an imaging device, allowing for inserting its head section into the device gantry, and providing reliable, safe and comfortable immobilization of the patient's head.

The invented system for transporting and immobilizing a patient, in particular stroke patient for CT imaging, comprises a stretcher made of rigid radiolucent material, with a body section and a narrower head section, and a removable head rest made of rigid radiolucent material and mounted immovably in the head section of the stretcher. The stretcher equipped with the head rest can be placed directly onto the table of the CT device, without a need to move the patient. Thus time required for the patient preparation for the CT examination as well as size of the medical team can be reduced.

The stretcher has externally flat, and internally flat or concave bottom. The body section of the stretcher has the body side walls. The head section of the stretcher has the head side walls and a head end wall which form a continuous vertical collar around the patient's head. This collar is joined to the body side walls of the stretcher. At the patient's feet side the stretcher is open thus enabling sliding the patient onto the stretcher, or from the stretcher onto the bed.

It is advisable that the collar composed of the head side walls and the head end wall is higher than the body side walls. Then the head rest can be firmly held down inside the head section of the stretcher.

The stretcher has the belt slits for fastening the immobilizing belts. The belt slits are shaped in the body side walls, and in the head side walls. The immobilizing belts comprise the velcro closures or plastic clips.

The stretcher has also the handle slits for transfer of the stretcher, shaped in the body side walls and in the bottom at the patient's feet side.

The stretcher can be made of polycarbonates, polystyrene, polypropylene, hardened polyethylene, or any other rigid and radiolucent polymeric material. The stretcher bottom is covered with an elastic liner suitable for sliding the patient onto the stretcher, or from the stretcher onto the bed.

The head rest which is part of the claimed system can be used together with a stretcher, when one needs to transport the patient or immobilize the patient's body. Alternatively, the head rest can be used alone, when one should only immobilize the patient's head. Then the head rest can be attached directly to the supporting device, e.g. to the table of the CT device. The head rest may be fastened to the supporting element with velcro closure.

The head rest consists of two parts: a head rest bottom part, and a head rest upper part. The head rest bottom part has a lower cavity for the back of the patient's head and neck, while the head rest upper part has an upper cavity for the facial part of the patient's head, with an opening for the patient's mouth, nose and eyes. The patient's head is placed on the head rest bottom part and covered with the head rest upper part. After immobilizing the patient's head, the head rest bottom part and the head rest upper part may be joined by a velcro closure or plastic clips. However, if the patient is placed on the stretcher, both parts of the head rest shall be secured in the head section with two immobilizing belts or bands anchored in the head side walls.

In one embodiment the cavities inside the head rest may be shaped individually for given patient. This is particularly advisable for radiotherapy within the head, where precise positioning of the beam relative to the target is crucial. However, the process of preparing individually shaped head rest takes too much time in emergency. Therefore the system is preferably equipped with a set of head rests shaped for various sizes of the scull.

In alternative embodiment the inner side walls of the lower cavity shaped in the head rest bottom part, and of the upper cavity shaped in the head rest upper part are vertical. Although the head is immobilized a bit less firmly, vertical inner walls enable to immobilize the head very quickly, even during transportation of the patient on the stretcher. The head rest is rather two-piece than monoblock, because it is simpler and safer to place the head on the bottom part and complete the head rest with the upper part, instead of putting the head inside a deep cavity in the monoblock head rest. However, the monoblock head rests are included in this invention.

Preferably the head rest is made of polymer foam, in particular polystyrene foam or polypropylene foam. The contour of the head rest has a cuboid shape fitting the head section of the stretcher.

An embodiment of the invented system for transporting and immobilizing a patient is shown schematically in the drawing, where:
Fig. 1 shows a general top view of the system with the patient placed inside and secured with the immobilizing belts;
Fig. 2 shows a top view of the system composed of the stretcher and the head rest;
Fig. 3 shows a side view I-I of the stretcher;
Fig. 4 shows a vertical longitudinal section A-A with the two-piece head rest fitting the head section;
Fig. 5 shows a vertical cross section B-B with the head rest fitting the head section of the stretcher and the patient's head;
Fig. 6 shows a vertical cross section C-C with the head rest fitting the head section of the stretcher and the patient's neck;
Fig. 7 shows a top view of the head section with inserted head rest bottom part;
Fig. 8 shows a vertical cross section D-D of the head section of the stretcher with inserted two-piece head rest;
Fig. 9 shows a vertical cross section E-E of the head section of the stretcher with inserted two-piece head rest;
Fig. 10 shows a perspective view of the head rest bottom part with vertical inner side walls;
Fig. 11 shows a perspective view of the head rest bottom and upper parts with vertical inner side walls.

The invented system for transporting and immobilizing a patient, in particular stroke patient for CT imaging, comprises the stretcher (1) made of rigid radiolucent material, with the body section (2) and narrower head section (3) which can be inserted into the gantry of the CT device. The head rest (4) made of rigid radiolucent material is mounted inside the head section (3) of the stretcher.

The bottom (5) of the stretcher (1) is externally flat and internally flat or concave. The body section (2) of the stretcher has the body side walls (6) preventing the patient from flalling down. There is no side wall at the foot side, thus enabling to slide the patient onto or from the stretcher. The head section (3) of the stretcher has the head side walls (7) and the head end wall (8) which are higher than the body side walls (6).

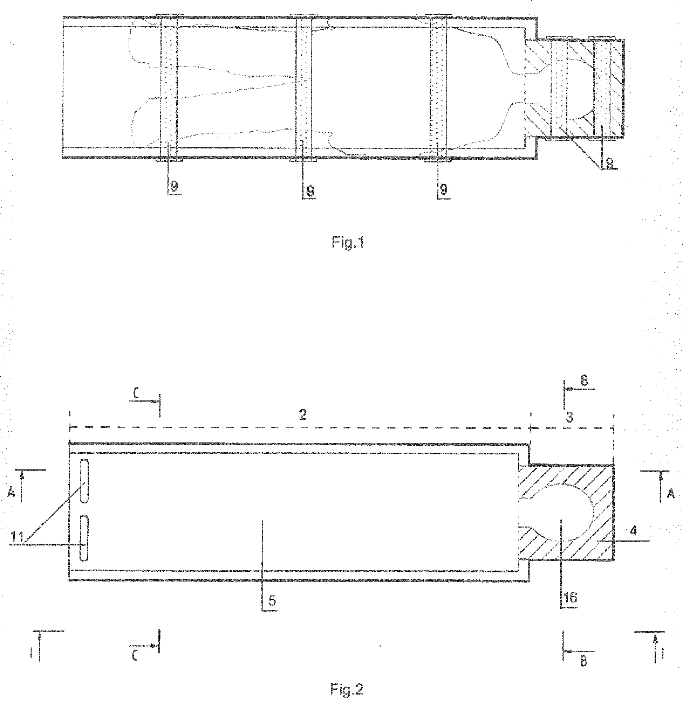

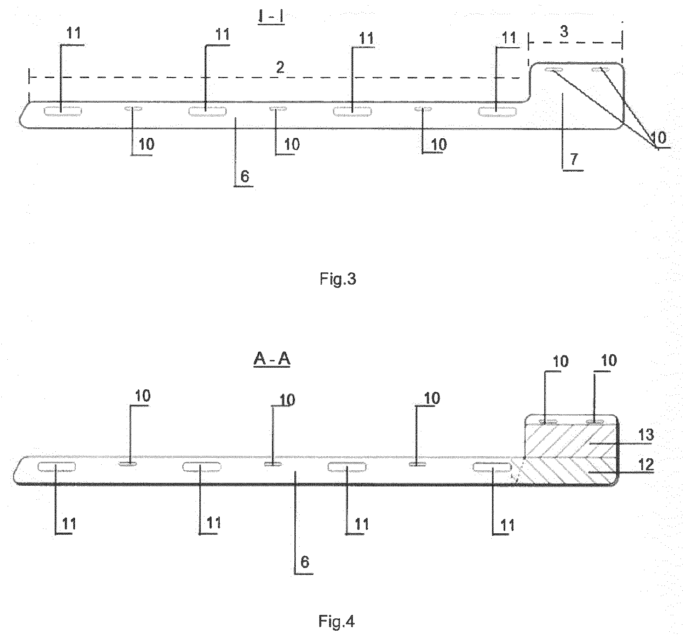

The stretcher (1) has the belt slits (10) for the immobilizing belts (9), shaped in the body side walls (6), and in the head side walls (7), as can be seen in Fig. 3, 4. The immobilizing belts (9) are fastened with the velcro closures or plastic clips. Fig. 1 shows the patient placed in the stretcher, with the head secured in the head rest, the body and head immobilized with the immobilizing belts.

The stretcher (1) also has the handle slits (11) for transfer of the stretcher (Fig. 3, 4), shaped in the body side walls (6) and in the bottom (5), close to the foot edge (Fig. 2). These handle slits in the bottom help to pull or push the stretcher.

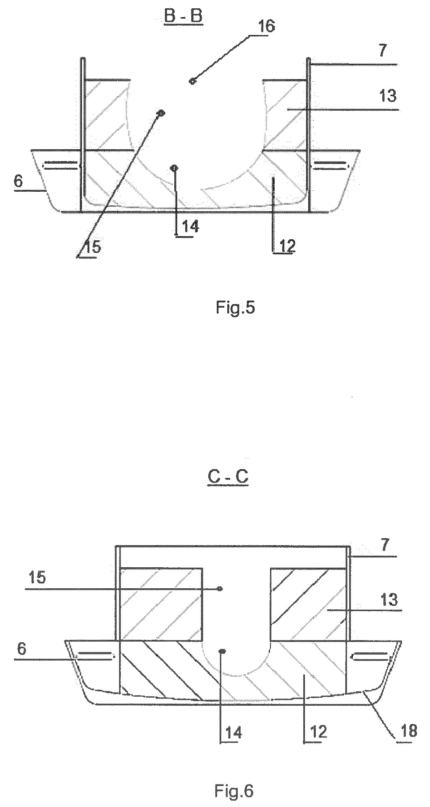

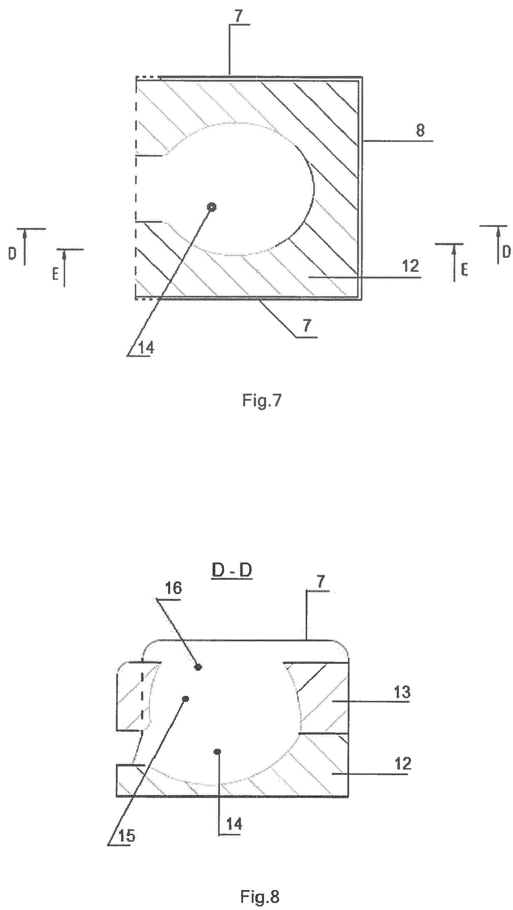

The head rest (4) has a cuboid shape which firmly fits the head section (3). The two-piece head rest (4) consists of the head rest bottom part (12), and the head rest upper part (13). The head rest bottom part (12) has the lower cavity (14) to receive the back of the patient's head and neck. The head rest upper part (13) has the upper cavity (15) shaped to receive the facial part of the patient's head, and has the opening (16) for the patient's mouth, nose and eyes. This is shown in Figs. 5-9. The head rest bottom part (12) and the head rest upper part (13) may be joined by a velcro closure or plastic clips, or may be secured in the head section (3) with the immobilizing belts or bands (9).

In one embodiment the head rest (4) is shaped individually for given patient.

Alternatively, the system comprises a set of head rests (4) shaped for various sizes of the scull.

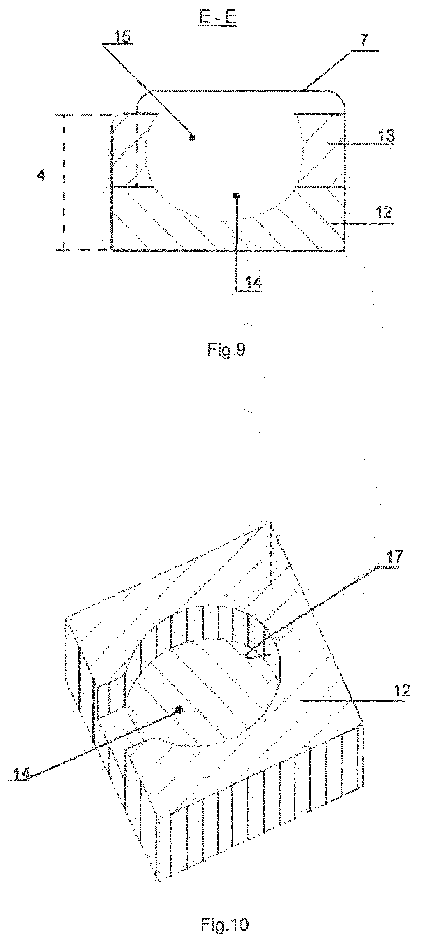

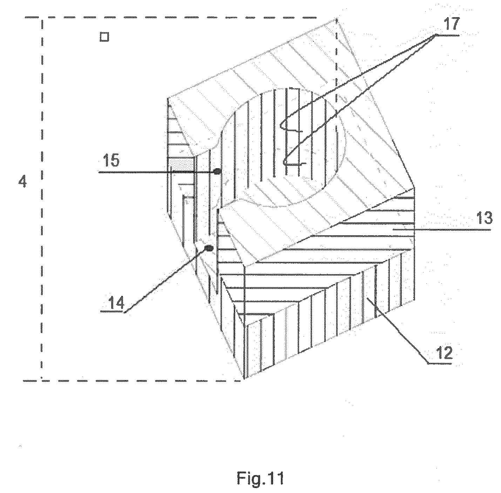

In another embodiment the inner side walls (17) of the lower cavity (14) shaped in the head rest bottom part (12), and of the upper cavity (15) shaped in the head rest upper part (13) are vertical, as is shown in Fig. 10, 11.

The head rest (4) is made of polymer foam, preferably polystyrene foam. Alternatively polypropylene foam may be used.

The stretcher (1) is made of polycarbonates, and its bottom (5) is covered with an elastic liner (18) suitable for sliding the patient from the stretcher onto the bed. The liner can be made of polyurethane foam, felt, rubber, etc. The liner can be fastened to the stretcher.

## Claims

1. A system for transporting and immobilizing a patient, in particular stroke patient for CT imaging, **characterized in that** it comprises a stretcher (1) made of rigid radiolucent material, with a body section (2) and a narrower head section (3), and a removable head rest (4) made of rigid radiolucent material and mounted in the head section of the stretcher.

2. The system according to claim 1, **characterized in that** the stretcher has externally flat and internally flat or concave bottom (5), and the body section (2) of the stretcher has the body side walls (6), and the head section (3) of the stretcher has the head side walls (7) and a head end wall (8).

3. The system according to claim 2, **characterized in that** the head side walls (7) and the head end wall (8) are higher than the body side walls (6)..

4. The system according to claim 2, or 3, **characterized in that** the stretcher (1) has the belt slits (10) for the immobilizing belts (9), shaped in the body side walls (6), and in the head side walls (7).

5. The system according to claim 2, or 3, or 4, **characterized in that** the stretcher (1) has the handle slits (11) for transfer of the stretcher, shaped in the body side walls (6) and in the bottom (5).

6. The system according to any of claims 1 to 5, **characterized in that** the head rest (4) has a cuboid shape and fits the head section (3).

7. The system according to any of claims 1 to 6, **characterized in that** the head rest (4) consists of a head rest bottom part (12), and a head rest upper part (13), wherein the head rest bottom part (12) has a lower cavity (14) for the back of the patient's head and neck, and the head rest upper part (13) has an upper cavity (15) for the facial part of the patient's head, with an opening (16) for the patient's mouth, nose and eyes.

8. The system according to any of claims 1 to 7, **characterized in that** the head rest (4) is shaped individually for given patient.

9. The system according to any of claims 1 to 7, **characterized in that** it comprises a set of head rests (4) shaped for various sizes of the scull.

10. The system according to claim 7, **characterized in that** the inner side walls (17) of the lower cavity (14) shaped in the head rest bottom part (12), and of the upper cavity (15) shaped in the head rest upper part (13) are vertical.

11. The system according to any of claims 1 to 10, **characterized in that** the head rest (4) is made of polymer foam, in particular polystyrene foam or polypropylene foam.

12. The system according to any of claims 7 to 11, **characterized in that** the head rest bottom part (12) and the head rest upper part (13) are joined by a velcro closure or plastic clips.

13. The system according to any of claims 1 to 12, **characterized in that** the stretcher (1) is made of polycarbonates.

14. The system according to any of claims 1 to 13, **characterized in that** the stretcher bottom (5) is covered with an elastic liner (18) suitable for sliding the patient from the stretcher onto the bed.

15. The system according to any of claims 4 to 14, **characterized in that** the immobilizing belts (9) comprise the velcro closures or plastic clips.

16. A head rest to immobilize the patient's head, in particular for CT imaging of the patient with stroke, **characterized in that** it is made of rigid radiolucent material and consists of a head rest bottom part (12), and a head rest upper part (13), wherein the head rest bottom part (12) has a lower cavity (14) for the back of the patient's head and neck, and the head rest upper part (13) has an upper cavity (15) for the facial part of the patient's head, with an opening (16) for the patient's mouth, nose and eyes.

17. The head rest according to claim 16, **characterized in that** the inner side walls (17) of the lower cavity (14) shaped in the head rest bottom part (12), and of the upper cavity (15) shaped in the head rest upper part (13) are vertical.

18. The head rest according to claim 16 or 17, **characterized in that** it is made of polymer foam, in particular polystyrene foam or polypropylene foam.

19. The head rest according to claim 16 or 17, or 18, **characterized in that** the head rest bottom part (12) and the head rest upper part (13) are joined by a velcro closure or plastic clips.

## Amended claims

### Amended claims in accordance with Rule 137(2) EPC.

1. A system for transporting and immobilizing a patient, in particular stroke patient for CT imaging, comprising a stretcher (1) having a body section (2) and a head section (3) provided with a removable head rest (4), all made of rigid radiolucent material, **characterized in that** external horizontal contours of the head section (3) and of the removable head rest (4) are narrower than external horizontal contour of the body section (2), and **in that** the system comprises a set of head rests (4) shaped for various sizes of the scull.

2. The system according to claim 1, **characterized in that** the stretcher has externally flat and internally flat or concave bottom (5), and the body section (2) of the stretcher has the body side walls (6), and the head section (3) of the stretcher has the head side walls (7) and a head end wall (8).

3. The system according to claim 2, **characterized in that** the head side walls (7) and the head end wall (8) are higher than the body side walls (6).

4. The system according to claim 2, or 3, **characterized in that** the stretcher (1) has the belt slits (10) for the immobilizing belts (9), shaped in the body side walls (6), and in the head side walls (7).

5. The system according to claim 2, or 3, or 4, **characterized in that** the stretcher (1) has the handle slits (11) for transfer of the stretcher, shaped in the body side walls (6) and in the bottom (5).

6. The system according to any of claims 1 to 5, **characterized in that** the head rest (4) has a cuboid shape and fits the head section (3).

7. The system according to any of claims 1 to 6, **characterized in that** the head rest (4) consists of a head rest bottom part (12), and a head rest upper part (13), wherein the head rest bottom part (12) has a lower cavity (14) for the back of the patient's head and neck, and the head rest upper part (13) has an upper cavity (15) for the facial part of the patient's head, with an opening (16) for the patient's mouth, nose and eyes.

8. The system according to any of claims 1 to 7, **characterized in that** the head rest (4) is shaped individually for given patient.

9. The system according to claim 7, **characterized in that** the inner side walls (17) of the lower cavity (14) shaped in the head rest bottom part (12), and of the upper cavity (15) shaped in the head rest upper part (13) are vertical.

10. The system according to any of claims 1 to 9, **characterized in that** the head rest (4) is made of polymer foam, in particular polystyrene foam or polypropylene foam.

11. The system according to any of claims 7 to 10, **characterized in that** the head rest bottom part (12) and the head rest upper part (13) are joined by a velcro closure or plastic clips.

12. The system according to any of claims 1 to 11, **characterized in that** the stretcher (1) is made of polycarbonates.

13. The system according to any of claims 1 to 12, **characterized in that** the stretcher bottom (5) is covered with an elastic liner (18) suitable for sliding the patient from the stretcher onto the bed.

14. The system according to any of claims 4 to 13, **characterized in that** the immobilizing belts (9) comprise the velcro closures or plastic clips.

15. A head rest made of rigid radiolucent material to immobilize the patient's head on a stretcher (1) having a body section (2) and a head section (3), as described in claims 1 to 14, in particular for CT imaging of the patient with stroke, **characterized in that** it consists of a head rest bottom part (12), and a head rest upper part (13), both having external horizontal contour narrower than external horizontal contour of the body section (2) of the stretcher (1), wherein the head rest bottom part (12) has a lower cavity (14) for the back of the patient's head and neck, and the head rest upper part (13) has an upper cavity (15) for the facial part of the patient's head, with an opening (16) for the patient's mouth, nose and eyes, and **in that** the head rests (4) are shaped for various sizes of the scull thus composing a system comprising a stretcher (1) and a set of the head rests (4).

16. The head rest according to claim 15, **characterized in that** the inner side walls (17) of the lower cavity (14) shaped in the head rest bottom part (12), and of the upper cavity (15) shaped in the head rest upper part (13) are vertical.

17. The head rest according to claim 15 or 16, **characterized in that** it is made of polymer foam, in particular polystyrene foam or polypropylene foam.

18. The head rest according to claim 15 or 16, or 17, **characterized in that** the head rest bottom part (12) and the head rest upper part (13) are joined by a velcro closure or plastic clips.
